(19) Europäisches Patentamt / European Patent Office / Office européen des brevets

(11) **EP 3 136 068 B1**

(12) **FASCICULE DE BREVET EUROPEEN**

(45) Date de publication et mention de la délivrance du brevet:
**17.07.2019 Bulletin 2019/29**

(51) Int Cl.:
**G01K 11/00** (2006.01)   **G01K 17/00** (2006.01)
**G01N 21/77** (2006.01)   **G02B 6/293** (2006.01)
**G01L 9/00** (2006.01)

(21) Numéro de dépôt: **16185772.7**

(22) Date de dépôt: **25.08.2016**

(54) **CAPTEUR DE FLUX THERMIQUE METTANT EN OEUVRE AU MOINS UN RESONATEUR OPTIQUE, CAPTEUR DE GAZ ET JAUGE PIRANI COMPORTANT AU MOINS UN TEL CAPTEUR**

WÄRMEFLUSSSENSOR, DER MINDESTENS EINEN OPTISCHEN RESONATOR EINSETZT, GASSENSOR UND PIRANI-VAKUUMMETER, DAS MINDESTENS EINEN SOLCHEN SENSOR UMFASST

HEAT-FLOW SENSOR USING AT LEAST ONE OPTICAL RESONATOR, GAS SENSOR AND PIRANI GAUGE COMPRISING AT LEAST ONE SUCH SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2015 FR 1558032**

(43) Date de publication de la demande:
**01.03.2017 Bulletin 2017/09**

(73) Titulaire: **Commissariat à l'énergie atomique et aux énergies alternatives**
**75015 Paris (FR)**

(72) Inventeur: **DURAFFOURG, Laurent**
**38500 VOIRON (FR)**

(74) Mandataire: **Brevalex**
**95, rue d'Amsterdam**
**75378 Paris Cedex 8 (FR)**

(56) Documents cités:
**US-A1- 2004 233 458     US-A1- 2007 269 901**
**US-A1- 2014 321 502     US-B1- 7 616 850**

• **David C Aveline ET AL: "Miniature Trace Gas Detector Based on Microfabricated Optical Resonators", Technology Focus: Sensors, 1 octobre 2013 (2013-10-01), pages 5-6, XP055281983, Extrait de l'Internet: URL:http://ntrs.nasa.gov/archive/nasa/casi .ntrs .nasa.gov/20140002259.pdf [extrait le 2016-06-20]**

**Description**

**DOMAINE TECHNIQUE ET ÉTAT DE LA TECHNIQUE ANTÉRIEURE**

La présente invention se rapporte à un capteur de flux thermique, qui peut être destiné à la mesure de la concentration d'un gaz ou à la mesure de très faibles pressions formant ainsi une jauge Pirani.

De façon générale, on entend par capteur de flux thermique, tout capteur mesurant un échange de chaleur entre le corps du capteur, par exemple sous forme de membrane, et le milieu fluidique dans lequel le capteur est disposé.

Un capteur de flux thermique est placé dans un environnement contenant l'élément à analyser, un analyte dans un gaz porteur dans le cas d'un capteur de gaz ou simplement un certain nombre de molécules de gaz dans le cas d'une jauge Pirani destinée à mesurer de faibles pressions.

Le document WO2011/044547 décrit un capteur TCD ("Thermal Conductivity Detectors") utilisant la variation de la conductivité thermique pour déterminer la composition de l'environnement gazeux dans lequel il est disposé. Celui-ci est disposé en sortie d'une colonne de chromatographie. Ce capteur TCD comporte une plaque support allongée, un élément chauffant situé sur la plaque support. La variation de tension est mesurée aux bornes de la plaque support chauffée pour déterminer la variation de résistance électrique qui dépend de la température de la plaque support et qui est représentative des échanges thermiques entre la plaque support et l'environnement gazeux; ces échanges thermiques dépendent de la composition de l'environnement gazeux.

Le document « A micro-Pirani vacuum gauge based on a micro-hotplate technology » F.T. Zhang et al, Sensors & Actuators A 126 (2006) 300-305 décrit une micro-jauge Pirani comportant une plaque suspendue par quatre poutre longues et deux poutres courtes et étroites. Une résistance chauffante et prévu sur la plaque.

Le capteur de gaz et la jauge Pirani présentant cette structure ont une limite de détection (LOD pour Limit of Détection en terminologie anglo-saxonne) qui reste supérieure à 1 ppm, notamment car la structure de ce capteur ainsi que celle de la micro-jauge Pirani ne permettent pas la mise en oeuvre d'une méthode de mesure dynamique, i.e. utilisant un signal d'entrée modulé, du fait de leur constante de temps thermique trop importante par rapport à la fréquence de modulation. Or, par exemple pour des applications biomédicales, pour un suivi de l'environnement ou de la qualité de l'air intérieur, on cherche à obtenir une limite de détection inférieure à 1 ppm.

Le document David C Aveline et al. « Miniature trace gas detector based on microfabricated optical resonators » Technology Focus : Sensors, octobre 2013, page 5-6 décrit un détecteur de gaz comportant un résonateur WGM disposé dans une enceinte sous vide et supporté par un élément de régulation de température.

**EXPOSÉ DE L'INVENTION**

C'est par conséquent un but de la présente invention d'offrir un capteur de flux thermique, notamment comme capteur de gaz ou comme jauge Pirani, pouvant présenter une limite de détection abaissée par rapport aux capteurs de l'état de la technique.

Le but énoncé ci-dessus est atteint par un capteur de flux thermique mettant en oeuvre au moins un résonateur optique, des moyens de chauffage du résonateur optique et des moyens pour mesurer la variation d'une caractéristique du résonateur optique en fonction de la variation de la composition de l'environnement dans lequel se trouve le capteur.

En effet, la température influe sur l'indice optique effectif du résonateur optique, la modification de cet indice modifie la fréquence de résonance du résonateur optique, qui peut être mesurée à partir de l'onde transmise ou réfléchie par le résonateur optique. On peut alors remonter à la variation de la conductivité thermique de l'environnement gazeux, par exemple du mélange gazeux, et donc à sa composition.

En d'autres termes, le capteur de flux thermique utilise un résonateur optique comme élément sensible aux échanges thermiques avec le milieu gazeux environnant.

Le résonateur optique est suspendu par des moyens de suspension, aptes à isoler thermiquement le résonateur optique.

Le capteur de flux thermique selon l'invention offre une limite de détection abaissée par rapport à celle des capteurs de l'état de la technique, un rapport 10 peut être atteint.

Une méthode de mesure utilisant un faisceau lumineux continu ou une méthode de mesure dynamique utilisant un faisceau modulé en amplitude peut être mise en oeuvre. Le capteur de flux thermique selon l'invention est particulièrement adapté à une méthode de mesure dynamique car le temps nécessaire à sa thermalisation est court, on parle de constante thermique faible. Le capteur offre alors une limite de détection encore abaissée. En outre, le capteur présente une gamme de mesure dynamique plus grande, i.e. il peut détecter des variations de température sur une large gamme, par exemple plusieurs dizaines de degrés.

Dans un mode de réalisation, l'échauffement du résonateur est obtenu par des moyens de chauffage de type à effet Joule situés à distance du résonateur optique.

Dans un autre mode de réalisation, l'échauffement du résonateur est un auto-échauffement obtenu en injectant un faisceau lumineux dans le résonateur optique d'une puissance suffisante pour échauffer le résonateur.

La présente invention a alors pour objet un capteur de flux thermique comportant au moins un résonateur optique suspendu à un support, ledit résonateur optique étant destiné à être suspendu dans un environnement gazeux, ledit résonateur optique comportant au moins un guide d'onde, au moins un premier moyen destiné à injecter un faisceau lumineux de mesure dans le guide d'onde du résonateur, au moins un deuxième moyen de collecte destiné à collecter un faisceau lumineux de détection provenant du guide d'onde du résonateur optique et des moyens de chauffage dudit résonateur optique.

Selon l'invention, les moyens de chauffage sont situés à distance du résonateur optique. La distance séparant les moyens de chauffage et le résonateur optique est par exemple comprise entre 200 nm et 10 $\mu$m.

Les moyens de chauffage peuvent être des moyens de chauffage par effet Joule. Dans un exemple de réalisation, les moyens de chauffage peuvent comporter au moins un fil conducteur suspendu audit support, le fil étant connecté à une source de tension électrique ou de courant électrique. Dans un autre exemple de réalisation, les moyens de chauffage comportent au moins une couche en matériau résistif électrique située en regard du résonateur optique et disposée sur le support, la couche en matériau résistif électrique étant connectée à une source de tension électrique ou de courant électrique.

Dans un autre mode de réalisation, les moyens de chauffage comportent une source de faisceau lumineux configurée pour injecter un faisceau lumineux dans le résonateur optique et dont la puissance assure un auto-échauffement du résonateur optique.

Selon une caractéristique additionnelle, le résonateur optique est suspendu au support par au moins une poutre. La poutre a par exemple une largeur comprise entre 50 nm et 10$\mu$m pour une épaisseur comprise entre 50 nm et 500 nm et une longueur comprise entre 1 $\mu$m et 100 $\mu$m.

Le résonateur optique peut comporter un anneau optique, un disque optique ou un cristal photonique.

Dans le cas d'un résonateur optique comportant un anneau, celui-ci peut avoir un rayon compris entre 1 $\mu$m et 100 $\mu$m.

Dans un exemple de réalisation, le premier moyen pour injecter un faisceau de lumière de mesure dans le résonateur optique est une guide d'onde et le deuxième moyen de collecte est formé par le même guide d'onde que celui du premier moyen, collectant un faisceau lumineux réfléchi.

Dans un autre exemple de réalisation, le premier moyen pour injecter un faisceau de lumière de mesure dans le résonateur optique est un guide d'onde, et deuxième moyen de collecte est formé par un autre guide d'onde, collectant un faisceau lumineux transmis.

Dans un autre exemple de réalisation, le premier moyen pour injecter un faisceau de lumière de mesure dans le résonateur optique est une guide d'onde, et le deuxième moyen de collecte collectant un faisceau lumineux réfléchi est formé par le guide d'onde du premier moyen, et dans lequel ledit capteur comporte également un autre guide d'onde collectant un faisceau lumineux transmis.

Dans la présenté demande, on entend par « faisceau transmis » un faisceau qui est collecté par un moyen de collecte, par exemple un guide d'onde, différent du moyen d'injection, par exemple un guide d'onde, et par « faisceau réfléchi » un faisceau qui est collecté par le moyen d'injection, par exemple par le guide d'onde d'injection.

La présente invention a également pour objet un capteur de gaz comportant au moins un capteur de flux thermique selon l'invention, le résonateur optique étant suspendu dans un canal fluidique entre une entrée d'alimentation en un mélange gazeux du canal fluidique et une sortie d'évacuation dudit mélange gazeux du canal fluidique.

La présente invention a également pour objet une jauge Pirani comportant au moins un capteur de flux thermique selon l'invention, le résonateur optique étant suspendu dans un volume milieu gazeux dont on souhaite mesurer la pression.

La présente invention a également pour objet un système de mesure comportant au moins capteur selon l'invention, un émetteur d'un faisceau lumineux de mesure connecté au premier moyen, au moins un photodétecteur connecté au deuxième moyen et une électronique de traitement des signaux émis par le photodétecteur.

L'émetteur d'un faisceau lumineux de mesure peut émettre un faisceau modulé en amplitude.

L'émetteur d'un faisceau lumineux de mesure est par exemple un laser.

Selon un exemple de réalisation, l'émetteur d'un faisceau lumineux de mesure forme également l'émetteur du faisceau lumineux permettant l'auto-échauffement du résonateur optique.

Le système de mesure peut comporter un autre capteur de gaz selon l'invention, le canal fluidique de l'un des capteurs étant destiné à être alimenté en mélange gazeux et le canal fluidique de l'autre capteur étant alimenté en gaz porteur uniquement, le deuxième moyen de collecte de chaque capteur étant connecté via un photodétecteur à l'électronique de traitement de sorte à permettre une mesure différentielle.

Le système de mesure peut comporter un autre capteur de gaz selon la l'invention, le canal fluidique de l'un des capteurs étant alimenté en mélange gazeux et le canal fluidique de l'autre capteur étant alimenté en gaz porteur uniquement, le deuxième moyen de collecte de chaque capteur étant connecté, via un dispositif de récupération et d'interférence des faisceaux lumineux de mesure issus des deux capteurs, à l'électronique de traitement de sorte à mesurer le déphasage entre les faisceaux de détection collectés par les deux deuxièmes moyens de sorte à permettre une mesure interférentielle.

## BRÈVE DESCRIPTION DES DESSINS

La présente invention sera mieux comprise sur la base de la description qui va suivre et des dessins en annexe sur lesquels:

- la figure 1 est une vue en perspective d'un exemple de réalisation d'un capteur de flux thermique selon un premier mode de réalisation,
- la figure 2 est une vue de dessus du capteur de la figure 1,
- la figure 3A est une vue en perspective d'un autre exemple de réalisation d'un capteur de flux thermique selon un premier mode de réalisation,
- la figure 3B est une vue de dessus du capteur de la figure 3A,
- la figure 4 est une représentation de la fonction de transfert d'un résonateur optique,
- la figure 5 est une vue en perspective d'un exemple de réalisation d'un capteur de flux thermique selon un deuxième mode de réalisation,
- la figure 6 est une représentation schématique d'un système de mesure comportant un capteur de flux thermique de la figure 3A,
- la figure 7 est une représentation schématique d'un système de mesure permettant une mesure différentielle,
- les figures 8A et 8B sont des représentations schématiques de systèmes de permettant une mesure interférentielle,
- la figure 9 est une représentation du circuit thermique équivalent du système formé par le support et le résonateur thermique.

## EXPOSÉ DÉTAILLÉ DE MODES DE RÉALISATION PARTICULIERS

Le capteur de flux thermique selon l'invention est destiné à être disposé dans un environnement gazeux par exemple, soit pour analyser la composition d'un mélange gazeux, soit pour mesurer un niveau de pression.

Dans les exemples qui vont suivre, le capteur de flux thermique est décrit dans son application à un capteur de gaz, il est donc destiné à être immergé dans un mélange gazeux à analyser s'écoulant dans le canal fluidique, le mélange gazeux comportant un gaz porteur tel que l'hélium et un ou des analytes à analyser.

Le capteur de flux thermique peut former une jauge Pirani, il peut par exemple être disposé dans une enceinte et permettre de surveiller son niveau de vide.

De manière générale, le capteur est disposé dans un volume de gaz.

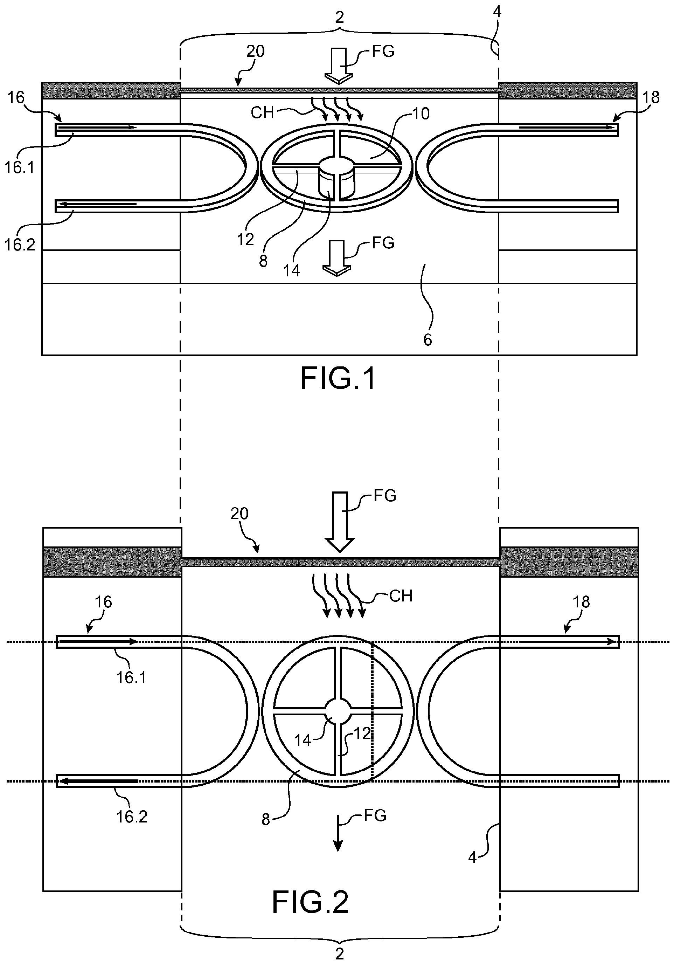

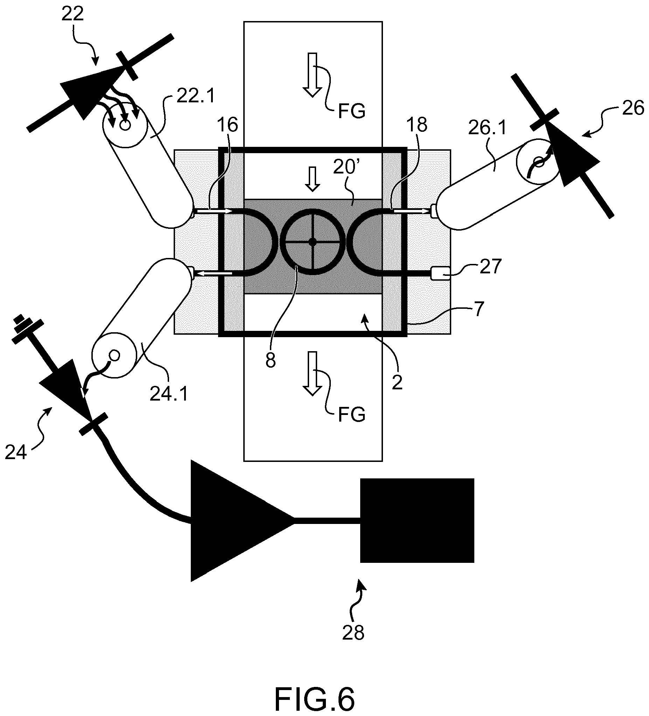

Sur les figures 1 et 2, on peut voir un exemple d'un capteur de flux thermique selon un premier mode de réalisation disposé dans un canal fluidique 2, le canal 2 comporte des parois latérales 4 reliées par une paroi transversale 6 formant le fond du canal 2. Le canal 2 est par exemple fermé par un capot 7 visible sur la figure 6.

Le canal 2 comporte une extrémité destinée à l'alimentation en gaz et une extrémité destinée à l'évacuation du gaz. Le flux gazeux est symbolisé par les flèches FG.

Le capteur comporte un résonateur optique 8 isolé thermiquement des parois du canal de sorte à limiter les fuites thermiques par diffusion vers le support, qui pourraient diminuer la sensibilité. Il est également de préférence isolé optiquement des parois du canal.

Le résonateur optique 8 est avantageusement suspendu dans le canal 2 par des moyens de suspension 10. Dans l'exemple représenté, le résonateur optique 8 comporte un guide d'onde formant un anneau optique. Les moyens de suspension mis en oeuvre comportent quatre bras 12 reliant le bord intérieur de l'anneau à un pion 14 solidaire du fond 6 du canal. La section des bras 12 est choisi suffisamment faible pour limiter les pertes thermiques, elle est par exemple comprise entre 1 nm et $1\mu m$. généralement les bras 12 sont dans le même matériau que le résonateur. En outre, le matériau du pion 14 est avantageusement choisi afin qu'il présente une faible conductivité thermique, par exemple il peut s'agir de $SiO_2$, du SiN, SiC... Pour limiter les pertes thermiques et assurer ainsi une bonne isolation thermique, le pion est avantageusement en oxyde, par exemple en en $SiO_2$ qui offre une faible conductivité thermique.

D'autres exemples de moyens de suspension sont envisageables, par exemple ils pourraient comporter des poutres s'étendant entre le bord extérieur du résonateur et les parois latérales du canal.

Le résonateur peut comporter un cristal photonique, par exemple en réalisant des trous dans le guide d'onde.

En variante, le résonateur optique peut comporter un guide d'onde formé par un disque optique ou un tore optique ou encore un cristal photonique, chacun étant suspendu par les moyens de suspension décrits ci-dessus.

De manière avantageuse, des trous de quelques dizaines de nanomètres de diamètres séparés de quelques dizaines de nanomètres sont percés dans les moyens de suspensions permettant à la fois une isolation thermique et optique du résonateur par rapport au support.

Le capteur comporte également au moins un guide d'onde 16 dont une partie au moins est disposée à proximité du résonateur optique afin d'injecter dans celui-ci un faisceau lumineux de mesure, dit faisceau sonde, et pour récupérer un faisceau lumineux transmis ou réfléchi, dit faisceau de détection.

Dans l'exemple représenté, le capteur comporte un premier guide d'onde 16 en forme du U, une branche 16.1 du U comporte une extrémité libre destinée à être reliée à une source de lumière et à injecter la lumière dans le résonateur optique 8 au niveau du fond du U. L'autre branche 16.2 du U collecte la lumière réfléchie par son extrémité libre par exemple destinée à être connectée à un photodétecteur.

Le capteur comporte un deuxième guide d'onde 18 qui est dans cet exemple également en forme de U destiné à collecter la lumière transmise par le résonateur. Le deuxième guide d'onde 18 est disposé à l'opposé du premier guide d'onde 16 par rapport au résonateur. Dans cet exemple les fonds du U sont en porte-à faux dans le canal pour être au plus près du résonateur.

Le capteur comporte également des moyens de chauffage 20 du résonateur optique, qui sont situés à distance du résonateur. Dans l'exemple représenté, il s'agit d'un fil conducteur électrique relié à une source de courant ou de tension. Le fil est par exemple en métal ou en matériau semi-conducteur, tel que le silicium. Dans cet exemple et de manière avantageuse, le fil est suspendu entre les deux parois latérales du canal transversalement au flux de gaz FG. De préférence, il est placé dans le plan contenant le résonateur optique.

La chaleur produite par le fil 20 est symbolisée par les flèches CH.

En variante, le fil chauffant pourrait s'étendre parallèlement au flux de gaz.

Dans l'exemple représenté, le fil est placé dans le plan du résonateur, il pourrait être disposé dans un plan parallèle et distinct du plan du résonateur, néanmoins cette réalisation est plus complexe.

Plusieurs fils conducteurs électriques pourraient être prévus

Le fil produit de la chaleur par effet Joule qui va chauffer le résonateur optique par conduction thermique et par rayonnement.

Par exemple, pour un canal de largeur et de hauteur typiquement comprises entre 10 $\mu$m et 200 $\mu$m, par exemple de largeur 100 $\mu$m et de hauteur 100$\mu$m, et un résonateur de diamètre compris entre 10$\mu$m et 100 $\mu$m, par exemple 50 $\mu$m, la distance entre le fil et le bord du résonateur est de l'ordre de quelques $\mu$m, par exemple comprise entre 200 nm à 10 $\mu$m.

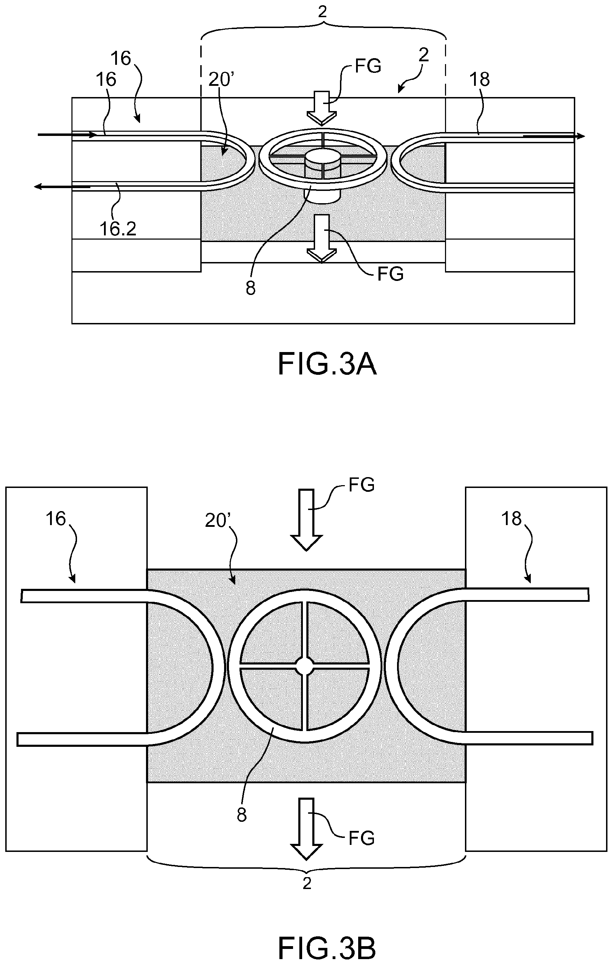

Sur les figures 3A et 3B, on peut voir un autre exemple de réalisation d'un capteur de flux thermique selon le premier mode de réalisation, dans lequel les moyens de chauffage sont formés par une couche résistive 20' disposée dans un plan parallèle au résonateur optique et à proximité du résonateur en considérant le flux gazeux. Dans la représentation de la figure 3A, la couche résistive est située sous le résonateur. La couche est déposée sur la paroi transversale du canal et est connectée à une source de tension ou de courant. La couche résistive présente avantageusement au moins une surface suffisante pour qu'une projection du résonateur dans le plan de la couche résistive soit inscrite dans la couche résistive 20'.

La couche résistive 20' est par exemple en TiN ou en W.

Cet exemple de réalisation présente l'avantage de chauffer plus uniformément le résonateur et plus rapidement. En outre, les températures atteintes peuvent être plus élevées, ce qui permet d'atteindre des limites de détection inférieure à celles du capteur des figures 1 et 2.

Dans les exemples décrits un seul résonateur optique est mis en oeuvre, mais on peut envisager d'utiliser plusieurs résonateurs couplés, par exemple en les disposant dans un même plan ou dans deux plans différents, par exemple deux plans parallèles, par exemple les plans étant parallèles à l'écoulement dans le canal. Par exemple deux anneaux résonants peuvent être couplés.

Sur la figure 6, on peut voir une représentation schématique d'un système de mesure selon l'invention comportant un capteur de flux thermique des figures 3A et 3B, un émetteur optique 22, par exemple une source laser, connecté à une entrée de la branche 16.1 du premier guide d'onde 16 et destiné à injecter de la lumière dans le résonateur, un premier photodétecteur 24, par exemple une photodiode, connecté à la sortie de la branche 16.2 du premier guide d'onde 16 pour collecter le faisceau réfléchi, un deuxième photodétecteur 26, par exemple une photodiode, connecté à la sortie du deuxième guide d'onde 18 pour collecter le faisceau transmis. Un réseau de couplage 27 est connecté à l'entrée du deuxième guide d'onde 18. Le réseau permet de coupler efficacement la lumière issue de la source, qui est généralement une source laser, dans le guide d'onde d'injection. Le même réseau permet aussi de découpler la lumière sortant du guide d'onde pour qu'elle soit collectée sur le photodétecteur.

On pourrait envisager d'injecter de la lumière dans les deux guides d'ondes 16 et 18 et de récupérer la lumière réfléchie dans les deux guides d'ondes 16 et 18 et de comparer les signaux obtenus.

L'entrée du canal 2 est par exemple connectée à la sortie d'une colonne de chromatographie en phase gazeuse ou d'un capillaire relié à une source de mélange gazeux.

L'émetteur optique 22 et les photodétecteurs 24, 26 sont par exemple connectés aux guides d'onde par des fibres optiques 22.1, 24, 26.1 respectivement.

Il est rappelé qu'un seul photodétecteur suffit, et que la collecte du faisceau transmis ou réfléchi est suffisante.

Les photodétecteurs 24 et 26 sont connectés à une électronique de traitement 28 qui détermine la composition du mélange gazeux à partir des signaux transmis par le ou les photodétecteurs.

Le fonctionnement du capteur de flux thermique des figures 1 et 2 utilisé comme capteur de gaz, va maintenant être décrit. Le fonctionnement du capteur des figures 3A et 3B est similaire à celui des figures 1 et 2.

Nous considérons un mélange gazeux comportant un gaz porteur par exemple l'hélium ou l'hydrogène, et au moins un analyte.

De préférence, on choisit un gaz porteur tel qu'il ait la propriété d'avoir un contraste thermique par rapport aux analytes important de façon à maximiser la différence entre la température avec le gaz porteur seul et la température en présence du mélange gaz porteur/analyte. Pour cela, on choisit par exemple un gaz porteur ayant une conductivité thermique k très supérieure à celles des analytes.

Dans le tableau ci-dessous sont rassemblées les valeurs de conductivité thermique de l'air et de l'hélium et deux analytes, le pentane et le benzène.

| Gaz | $H_2$ | Hélium | Air | Pentane | Benzène |
|---|---|---|---|---|---|
| $kgaz(mW/m.K)$ | 168 | 143 | 24 | 15 | 8 |

Lors d'une première étape, le mélange gazeux circule dans le canal autour du résonateur. Le fil est alimenté en courant ou en tension continue, le résonateur optique est alors chauffé par conduction thermique et rayonnement. Le mélange gazeux a une composition stable. Un équilibre thermique s'installe, le résonateur a alors une température T.

En considérant la composition du mélange gazeux dans le canal stable, le résonateur est échauffé à une température donnée qui résulte d'un équilibre thermique entre le flux de chaleur provenant du fil chauffant par conduction et rayonnement et des pertes thermiques par échange avec le support formant thermostat à travers le mélange gazeux et à travers les moyens de suspension.

La puissance délivrée $P_J$ par le fil 20 par effet Joule s'écrit :

$$P_J = R_0 I^2$$

$R_0$ étant la résistance électrique du fil conducteur 20, I l'intensité électrique du courant le traversant.

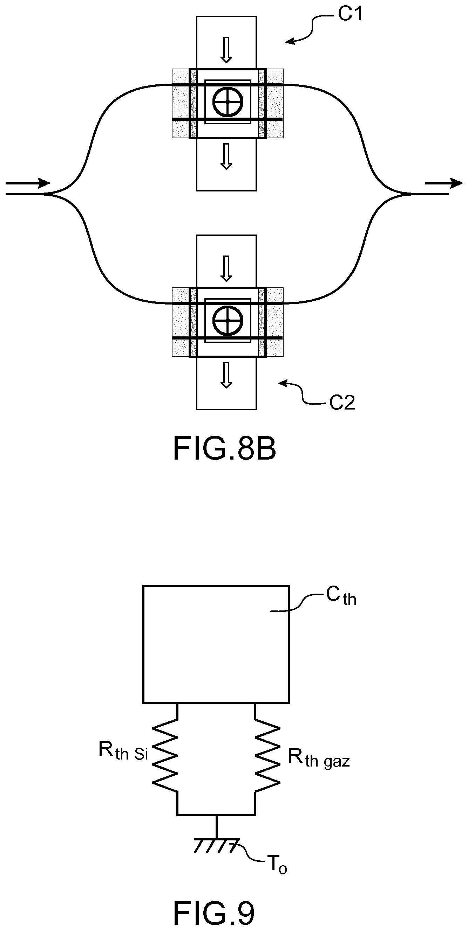

Le résonateur optique chauffé peut s'assimiler à une capacité thermique $C_{th}$ connectée à un thermostat par le biais d'une résistance thermique comme cela est schématisé sur la figure 9. Le résonateur optique est entouré d'un gaz.

La résistance thermique $R_{th}$ caractérisant les échanges entre le résonateur et l'extérieur est composée de la résistance thermique de l'environnement gazeux $R_{th\text{-}gaz}$ et la résistance thermique des bras de suspension du résonateur par exemple en silicium $R_{th\text{-}Si}$.

A l'équilibre thermique, le résonateur optique est à une température stable T qui s'écrit :

$$T = T_0 + P.R_{th} \text{ (I)}$$

$T_0$ est la température des parois du canal et celle du résonateur optique en l'absence de chauffage.

En considérant un mélange gazeux contenant le gaz porteur et un analyte, la conductivité thermique du mélange gazeux s'écrit :

$$\kappa gaz = \kappa 1 c + \kappa 2 (1-c) \ (W.m^{-1}.K^{-1}) \text{ (III)}$$

Avec k1 la conductivité thermique de l'analyte,
C la concentration en analyte
k2 la conductivité thermique du gaz porteur.

Pour information, la variation relative de la conductivité thermique est de 1 ppm pour 1 ppm de variation relative de la concentration en analyte dans le gaz porteur.

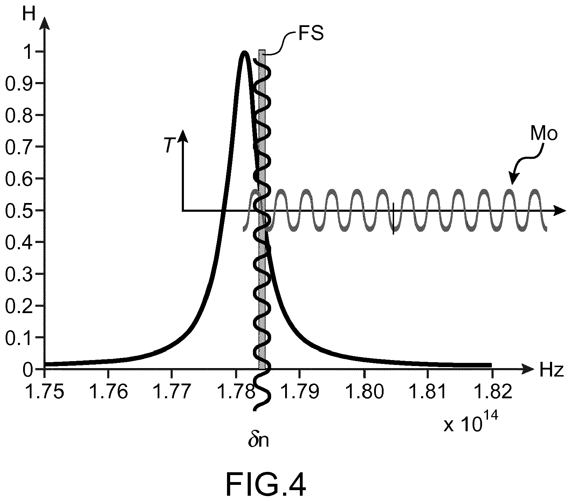

Un faisceau sonde, par exemple continu, est injecté dans le résonateur via le premier guide d'onde. De préférence la longueur d'onde du faisceau sonde (ou sa fréquence $F_s$) est décalée par rapport à la longueur d'onde correspondant au maximum de transmission ou de réflexion comme cela est schématisé sur la représentation de la fonction de transfert H d'un résonateur optique en fonction de la fréquence en Hz sur la figure 4. Un faisceau sonde modulée en amplitude peut également être injecté dans le résonateur pour réaliser une mesure dynamique comme cela sera décrit ci-dessous.

Lors d'une deuxième étape, la concentration en analyte varie.

Au vu de la relation (III), la conductivité thermique du mélange gazeux varie, les échanges thermiques entre le résonateur optique et le mélange gazeux sont modifiées ce qui modifie l'équilibre thermique et la température du résonateur thermique.

La variation de température du résonateur optique est estimée avec l'équation de diffusion de la chaleur à travers ce mélange et dans les encastrements tenant le résonateur optique la variation de température peut s'écrire :

$$\Delta T = P/G$$

P étant la puissance dissipée amenée par effet Joule, $G$ représentant la conductance thermique du système et contient un terme relatif aux échanges par le mélange gazeux et un terme relatif aux échanges par le matériau formant les moyens de suspension et le pion, le cas échéant, les autres modes de dissipation étant considérés comme négligeables : $G$ peut alors s'écrire G = $Ggaz$+$Gs$, Gs désignant la conductance du silicium formant le résonateur et les moyens de suspension.

Avec

$$Ggaz = \kappa gaz(S/g) \times \gamma$$

$Ggaz$ est calculé en considérant deux plans en regard, par exemple un plan contenant le résonateur tel qu'un disque, un anneau ou un cristal photonique en regard du substrat placé dessous. S est la surface équivalente du résonateur. $\delta$ est un facteur correctif sans unité tenant compte du fait que la diffusion ne se fait pas simplement par des flux perpendiculaires. Ce coefficient dépend en plus de la distance g entre les deux plans. En outre, comme cela a été mentionné préalablement, de préférence le capteur est tel que la conductance thermique à travers les suspensions du résonateur ou l'encastrement du résonateur est faible par rapport à la conductance à travers le gaz. En considérant des suspensions fines et le pion en matériau isolant thermique, le rapport Gs/Ggaz peut être inférieur à 1%, le terme de conduction à travers l'encastrement devient donc négligeable.

La fonction de transfert de la variation de température en fonction de la variation de conductivité, $\delta T \delta \kappa gaz$/autour d'une concentration initiale $c_0$ peut s'écrire

$$\partial \Delta T/\partial \kappa gaz \sim (gP/S\gamma \kappa^2{}_{gaz})_{T=T0,c=c0}$$

Sa valeur dépend de la valeur de la distance g et de la puissance injectée pour chauffer le résonateur optique. Par exemple en considérant g de l'ordre de 1 $\mu$m. Cette expression mène à une valeur typique de la variation relative de la température en fonction de la conductivité :

$$\kappa gaz/\Delta T \times \partial \Delta T/\partial \kappa gaz \sim 1$$

Cette variation de température du résonateur provoque une variation de l'indice optique effectif du résonateur.

Par conséquent en chauffant le résonateur optique, l'indice optique effectif est modifiée, la valeur à l'équilibre thermique est désignée « valeur nominale »

La variation d'indice optique en fonction de la température dépend de la longueur d'onde et de la température $T_0$, qui est le point de fonctionnement thermique correspondant à la température avec le gaz porteur uniquement (sans analyte).

Lorsque la composition du mélange gazeux change, la conductivité thermique du mélange gazeux change ce qui implique que les échanges thermiques entre les moyens de chauffage et le résonateur optique et entre le résonateur optique et le support sont modifiés. L'équilibre thermique est modifié et la température du résonateur augmente ou diminue, l'indice optique effectif subit une variation $\delta n$ autour d'une valeur nominale qui est schématisée sur la représentation de la fonction de transfert de la figure 4.

Or la fréquence de résonance optique du résonateur dépend de l'indice optique effectif. La modification de l'indice optique effectif provoque donc une variation de la fréquence de résonance optique du résonateur.

On peut estimer l'effet global sur la fréquence de résonance optique par la variation relative de la fréquence de résonance en fonction de la température :

$$1/vR \times \partial v_R/\partial T \sim 50 ppm$$

Il est à noter que la variation de la fréquence de résonance du résonateur a plusieurs origines qui sont notamment la variation de l'indice effectif, la variation de la longueur de propagation due à la dilatation du résonateur et à la variation des contraintes thermiques de la couche. Or, par exemple dans le cas du silicium, le coefficient de dilatation typique du silicium est de 3 ppm et la variation de l'indice effective d'un guide silicium pour une longueur d'onde 1,55 $\mu$m est de l'ordre de 50 ppm. Au premier ordre, la variation de l'indice effectif est donc l'effet prépondérant. Dans la suite de la description, on considérera donc que la variation de l'indice optique effectif est principalement la cause de la variation de la fréquence de résonance du résonateur.

La fonction de transfert se déplace donc le long de l'axe des abscisses en fonction de la température du résonateur optique, i.e. la fréquence de résonance qui correspond au maximum du pic varie. Cette variation se traduit par une modulation de l'amplitude de l'intensité lumineuse de détection qui est le signal transmis ou réfléchi. Cette variation de fréquence de résonance module l'intensité optique transmise ou réfléchie par le résonateur.

Cette modulation Mo est schématisée sur la fonction de transfert de la figure 4.

En mesurant cette modulation il est possible de remonter au décalage de la fréquence de résonance optique et à l'intensité optique transmise ou réfléchie. Il est alors possible de déterminer la conductivité du mélange gazeux. Plus le facteur de qualité du résonateur optique est élevé, plus la variation d'intensité lumineuse sera importante. Et connaissant le composants du mélange gazeux et leurs conductivités thermiques, il est possible de remonter à la composition du mélange gazeux, i.e. à la variation de la concentration du ou des analytes.

En limitant les pertes thermiques par les moyens de suspension, on s'assure que la principale cause de la variation de la température est due à la variation de la composition du mélange gazeux.

Le capteur de flux thermique selon l'invention présentant une constante thermique très faible, il permet alors très avantageusement de mettre en oeuvre une méthode de mesure dynamique. On peut alors exploiter une détection synchrone en modulant l'intensité de la lumière injectée. Dans ce cas, un amplificateur à détection synchrone est utilisé pour démoduler le signal issu des photodiodes. Cela permet de minimiser l'impact du bruit en 1/f et d'avoir un rapport entre le signal et le bruit de fond continu plus important. La limite de détection du capteur selon l'invention peut encore être abaissée. En outre, il présente une gamme dynamique plus importante.

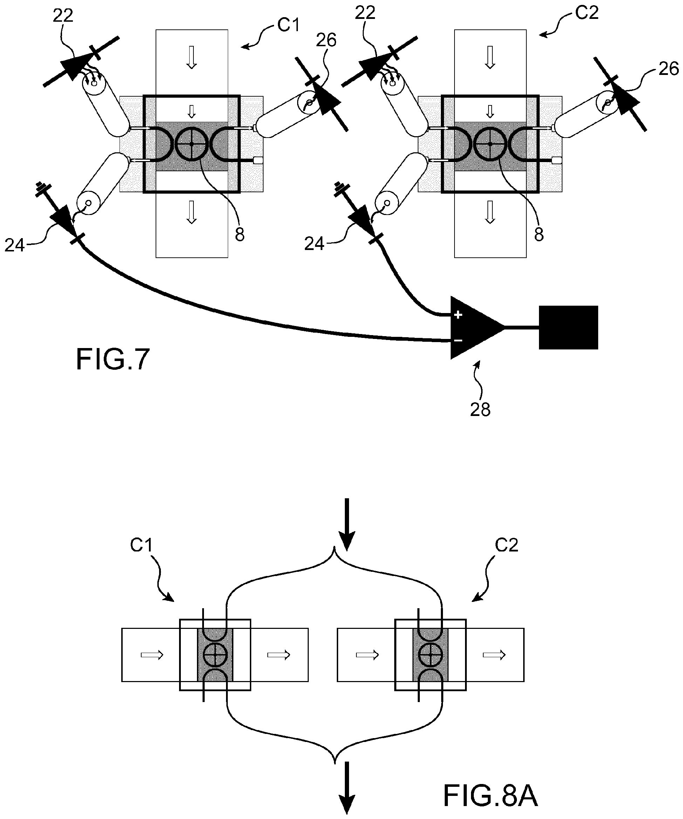

Sur la figure 7, on peut voir un exemple de réalisation d'un système permettant une mesure différentielle.

Le système comporte deux capteurs de flux thermique selon l'invention C1 et C2 chacun disposant de son propre canal fluidique. Le système comporte un émetteur optique pour chaque capteur ou un émetteur pour les deux capteurs et un ou des photodétecteurs pour chacune des capteurs.

Le canal fluidique du capteur C1 est uniquement alimenté en gaz porteur et le canal fluidique du capteur C2 est alimenté en gaz porteur uniquement ou en mélange gazeux à analyser (gaz porteur et analyte).

Les photodétecteurs des deux capteurs sont connectés à la même électronique de traitement. On peut mesurer ainsi la différence de signaux électriques obtenus par les photodétecteurs des deux capteurs, ce qui permet de supprimer le bruit de fond continu et de limiter les dérives thermiques au premier ordre.

Sur les figures 8A et 8B, on peut voir un autre exemple de système de mesure mettant en oeuvre avantageusement une méthode interférentielle dans une structure interférentielle par exemple de type Mach-Zhender.

Comme pour le système de la figure 7, le système de mesure comporte deux capteurs de flux thermique selon l'invention C1 et C2 chacun disposant de son propre canal fluidique. Le système comporte un émetteur optique 22 pour les deux capteurs et un photodétecteurs collectant la lumière détectée après interférence.

Le canal fluidique du capteur C1 est uniquement alimenté en gaz porteur et le canal fluidique du capteur C2 est alimenté soit en gaz porteur uniquement, soit en mélange gazeux à analyser (gaz porteur et analyte).

Les faisceaux transmis collectés dans les deux capteurs sont combinés et c'est le signal résultant qui est traité par l'électronique.

Sur la figure 8A, les capteurs sont du type de ceux la figure 3A et 3B et sur la figure 8B, les capteurs comportent des guides d'onde droits. Par ailleurs, la structure interférentielle comporte dans cet exemple des guides d'onde de récupération des faisceaux lumineux de mesure, issus des deux capteurs. Ces guides d'ondes sont montés en « Y » pour permettre auxdits faisceaux de mesure d'interférer entre eux. Bien entendu, tout autre dispositif de récupération et d'interférence des faisceaux lumineux de mesure issus des deux capteurs, peut être utilisé à la place des guides d'ondes montés en « Y ».

Dans ce système, on mesure le déphasage d'une branche d'un Mach-Zhender par rapport à une branche de l'autre Mach-Zhender, et non plus l'amplitude du signal.

En variante, le système peut faire interférer les faisceaux réfléchis.

Le système interférentiel étant un mode différentiel entièrement optique, une électronique plus simple peut être utilisée et un système plus compact peut ainsi être réalisé.

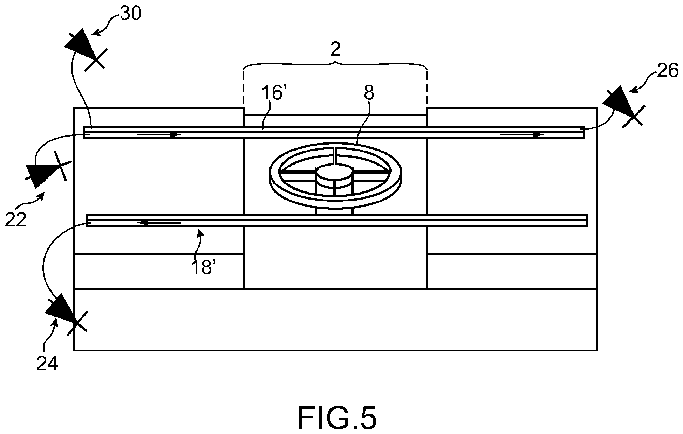

Sur la figure 5, on peut voir un exemple de réalisation d'un capteur selon un deuxième mode de réalisation. Ce capteur diffère des capteurs du premier mode de réalisation par le fait qu'il ne comporte pas de moyens de chauffage externe.

Le capteur comporte une source de lumière 30 connectée au premier guide d'onde 16' qui peut assurer un auto-échauffement du résonateur optique 8 suspendu dans le canal 2. Le guide d'onde 16' collecte la lumière transmise et le guide d'onde 18' collecte la lumière réfléchie.

Dans cet exemple de réalisation les guides 16' et 18' sont parallèles et traversent le canal. On pourrait prévoir des guides d'onde identiques aux guides d'onde 16 et 18.

Dans l'exemple représenté, il s'agit d'une source de lumière supplémentaire, par rapport à la source sonde 22, qui envoie dans le premier guide d'onde 16 un faisceau lumineux d'une puissance suffisamment forte. Le mécanisme d'absorption transforme cette énergie optique en énergie thermique chauffant le résonateur.

Les échauffements locaux peuvent aller de quelques degrés pour des puissances faibles, par exemple inférieures à 100μW à plusieurs centaines de degrés pour de fortes puissances, par exemple de l'ordre de la dizaine de mWatt ou de quelques dizaines de mWatts.

La source de lumière supplémentaire 30 est par exemple un laser dont la longueur d'onde d'émission peut être différente de la longueur d'onde de la source sonde.

En variante, on peut utiliser une seule source de lumière à la fois injectant le faisceau sonde pour la mesure et chauffant le résonateur.

Dans ce mode de réalisation, lorsque la conductivité thermique du mélange gazeux entourant le résonateur varie, seuls les échanges thermiques entre le résonateur et le support formé par le canal sont affectés puisque l'échauffement est interne au résonateur.

Le principe de fonctionnement du capteur selon le deuxième mode de réalisation est similaire à celui selon le premier mode de réalisation.

La température T du résonateur résulte d'un équilibre thermique entre l'augmentation de température et les échanges thermiques avec l'environnement gazeux et peut s'écrire :

$$T = T_0 + \Delta T = T_0 + P_{opt}/(G_{th\_Si} + G_{th\_gaz})$$

$T_0$ étant la température initiale du résonateur

$P_{opt}$ étant la puissance délivrée par la source lumineuse de chauffage.

$G_{th\_Si}$ et $G_{th\_gaz}$ étant les conductances thermiques du silicium et du gaz respectivement. Gth_Si permet de caractériser les échanges à travers les moyens de suspension.

On mesure la variation de la fréquence de résonance optique du résonateur. Elle varie en fonction de la température du résonateur qui dépend des échanges avec l'environnement gazeux et donc de sa composition.

Nous allons estimer les performances du capteur de mesure selon l'invention. En considérant que la limite inférieure de résolution est donnée par la plus petite variation spectrale mesurable correspondant à la largeur à mi-hauteur d'un pic de la réponse en transmission ou en réflexion et en considérant un micro résonateur en silicium avec une finesse $F$= 1000, d'indice effectif $neff$=2.4, et de rayon $R$=50μm, soit un intervalle spectral libre ou $ISL$ de l'ordre de 3,2 nm. La largeur à mi-hauteur sera donc de l'ordre de 3,2 pm, ce qui correspond à un facteur de qualité de $10^5$. La sensibilité de la longueur d'onde de résonance à la température étant de l'ordre de 75 ppm/°C (soit ~50 ppm pour une longueur d'onde sonde de 1,55 μm), la résolution en température théorique est de l'ordre de 0,05°K (soit : $\delta\Delta T$ = 0,05°).

Pour une température de fonctionnement de 200°C, on détermine que la plus petite variation de concentration mesurable est de 200 ppb à 1000 ppm. La limite de détection absolue dépend du couple gaz porteur et analyte. Dans le cas du benzène dans de l'hélium, la limite de détection est de l'ordre de 7ppm. Les TCD de l'état de la technique permettent de détecter des variations de concentration du benzène de 10ppm à quelques dizaines de ppm, voire une centaine de ppm.

Il est à noter que ce calcul ne tient pas compte des bruits inhérents à la source (bruit de photons) et au détecteur (bruit de Grenaille, $1/f$, ...) mais seulement de la finesse spectrale du résonateur optique.

Des valeurs de dimensionnement vont être données à titre d'exemple uniquement.

La largeur du guide d'onde peut être comprise entre 100 nm et 500 nm pour une hauteur comprise par exemple entre 100 nm et 500 nm. Le guide d'onde a par exemple les dimensions suivantes 500 nmx200 nm.

La distance entre un guide d'onde et le résonateur optique est par exemple comprise entre 50 nm et 500 nm, par exemple égale à 200 nm.

La distance entre l'élément chauffant et le résonateur est de l'ordre de 1 µm et peut atteindre plusieurs microns, elle est par exemple égale à 2 µm.

Dans le cas d'un résonateur optique de type anneau, son rayon peut être compris entre 1 µm à 100 µm, et par exemple égal à 50 µm.

Par exemple, le ou les résonateurs optiques peuvent être réalisés en silicium, en silicium dopé, en nitrure de silicium, en niobate de lithium ou en des matériaux III/V à base de GaAs. La longueur d'onde du ou des lasers formant les émetteurs est adaptée en fonction des matériaux et des dimensions des guides, notamment de leur section et en fonction du rayon des résonateurs en anneau ou disque. Par exemple, dans le cas d'un résonateur optique en silicium ou en des matériaux III/V la longueur d'onde est 1,55 µm, dans le cas d'un résonateur optique en alliage SiGe, la gamme de longueur d'onde va de 3 µm à 7 µm environ, dans le cas d'un résonateur optique en Ge les longueurs d'onde seront dans une gamme 7 µm - 12µm.

Le capteur thermique peut être réalisé par des méthodes connues de la microélectronique par exemple à partir d'un substrat SOI (Silicon on Insulator), en appliquant notamment des étapes de lithographie, gravure, croissance par épitaxie.

## Revendications

1. Capteur de flux thermique comportant au moins un résonateur optique (8), un support, ledit résonateur optique (8) comportant au moins un guide d'onde, au moins un premier moyen (16) destiné à injecter un faisceau lumineux de mesure dans le guide d'onde du résonateur optique, au moins un deuxième moyen de collecte (16, 18) destiné à collecter un faisceau lumineux de détection provenant du guide d'onde du résonateur optique et des moyens de chauffage (20, 20', 30) dudit résonateur optique (8) **caractérisé en ce que** le guide d'onde est suspendu au support par des moyens de suspension isolant thermiquement le résonateur et **en ce que** ledit résonateur optique (8) est destiné à être suspendu dans un environnement gazeux, et **en ce que** les moyens de chauffage (20) sont situés à distance du résonateur optique (8).

2. Capteur de flux thermique selon la revendication 1, dans lequel la distance séparant les moyens de chauffage (20) et le résonateur optique (8) est comprise entre 200 nm et 10 µm.

3. Capteur de flux thermique selon la revendication 1 ou 2, dans lequel les moyens de chauffage (20) sont des moyens de chauffage par effet Joule.

4. Capteur de flux thermique selon la revendication 1, 2 ou 3, dans lequel les moyens de chauffage (20) comportent au moins un fil conducteur suspendu audit support, le fil étant connecté à une source de tension électrique ou de courant électrique ou dans lequel les moyens de chauffage (20') comportent au moins une couche en matériau résistif électrique située en regard du résonateur optique (8) et disposée sur le support, la couche en matériau résistif électrique étant connectée à une source de tension électrique ou de courant électrique.

5. Capteur de flux thermique selon la revendication 1, dans lequel les moyens de chauffage (30) comportent une source de faisceau lumineux configurée pour injecter un faisceau lumineux dans le résonateur optique (8) et dont la puissance assure un auto-échauffement du résonateur optique (8).

6. Capteur de flux thermique selon l'une des revendications 1 à 5, dans lequel les moyens de suspension comportent au moins une poutre (12), la poutre (12) ayant avantageusement une largeur comprise entre 50 nm et 10 µm pour une épaisseur comprise entre 50 nm et 500 nm et une longueur comprise entre 1 µm et 100 µm.

7. Capteur de flux thermique selon l'une des revendications 1 à 6, dans lequel le résonateur optique (8) comporte un disque optique, un cristal photonique ou un anneau optique de rayon par exemple compris entre 1 µm et 100 µm

8. Capteur de flux thermique selon l'une des revendications 1 à 7, dans lequel le premier moyen pour injecter un faisceau de lumière de mesure dans le résonateur optique (8) est une guide d'onde et le deuxième moyen de collecte est formé par le même guide d'onde que celui du premier moyen, collectant un faisceau lumineux réfléchi.

9. Capteur de flux thermique selon l'une des revendications 1 à 7, dans lequel le premier moyen pour injecter un faisceau de lumière de mesure dans le résonateur optique (8) est un guide d'onde, et le deuxième moyen de collecte est formé par un autre guide d'onde, collectant un faisceau lumineux transmis.

**10.** Capteur de flux thermique selon l'une des revendications 1 à 7, dans lequel le premier moyen pour injecter un faisceau de lumière de mesure dans le résonateur optique (8) est une guide d'onde, et le deuxième moyen de collecte collectant un faisceau lumineux réfléchi est formé par le guide d'onde du premier moyen, et dans lequel ledit capteur comporte également un autre guide d'onde (18) collectant un faisceau lumineux transmis.

**11.** Capteur de gaz, comportant au moins un capteur de flux thermique selon l'une des revendications 1 à 10, dans lequel le résonateur optique (8) est suspendu dans un canal fluidique (2) entre une entrée d'alimentation en un mélange gazeux du canal fluidique et une sortie d'évacuation dudit mélange gazeux du canal fluidique (2).

**12.** Capteur formant jauge Pirani comportant au moins un capteur de flux thermique selon l'une des revendications 1 à 10, dans lequel le résonateur optique (8) est suspendu dans un milieu gazeux dont on souhaite mesurer la pression.

**13.** Système de mesure comportant au moins capteur selon l'une des revendications 1 à 12, un émetteur (22) d'un faisceau lumineux de mesure connecté au premier moyen, au moins un photodétecteur (24) connecté au deuxième moyen et une électronique (28) de traitement des signaux émis par le photodétecteur, l'émetteur (22) émettant avantageusement un faisceau modulé en amplitude, et/ou dans lequel l'émetteur (22) est un laser.

**14.** Système de mesure selon la revendication 13 en combinaison avec la revendication 5, dans lequel l'émetteur (22) forme également l'émetteur du faisceau lumineux permettant l'auto-échauffement du résonateur optique

**15.** Système de mesure selon la revendication 13 ou 14, comportant un autre capteur de gaz selon la revendication 11, dans lequel le mélange gazeux comporte un gaz porteur et au moins un analyte à détecter, le canal fluidique de l'un des capteurs de gaz (C2) étant destiné à être alimenté en mélange gazeux et le canal fluidique de l'autre capteur de gaz (C1) étant alimenté en gaz porteur uniquement, le deuxième moyen de collecte de chaque capteur de gaz étant connecté via un photodétecteur à l'électronique de traitement de sorte à permettre une mesure différentielle.

**16.** Système de mesure comportant selon la revendication 13 ou 14, comportant un autre capteur de gaz selon la revendication 11 et dans lequel le mélange gazeux comporte un gaz porteur et au moins un analyte à détecter, le canal fluidique de l'un des capteurs de gaz (C2) étant alimenté en mélange gazeux et le canal fluidique de l'autre capteur de gaz (C1) étant alimenté en gaz porteur uniquement, le deuxième moyen de collecte de chaque capteur de gaz étant connecté, via un dispositif de récupération et d'interférence des faisceaux lumineux de mesure issus des deux capteurs de gaz, à l'électronique de traitement de sorte à mesurer le déphasage entre les faisceaux de détection collectés par les deux deuxièmes moyens de sorte à permettre une mesure interférentielle.

**Patentansprüche**

**1.** Wärmeflusssensor, umfassend wenigstens einen optischen Resonator (8), einen Träger, wobei der optische Resonator (8) mindestens einen Wellenleiter umfasst, wenigstens ein erstes Mittel (16), welches dazu bestimmt ist, einen Messlichtstrahl in den Wellenleiter des optischen Resonators zu injizieren, wenigstens ein zweites Mittel zum Sammeln (16, 18), welches dazu bestimmt ist, einen Detektionslichtstrahl zu sammeln, der von dem Wellenleiter des optischen Resonators kommt, und Heizmittel (20, 20', 30) des optischen Resonators (8), **dadurch gekennzeichnet, dass** der Wellenleiter an dem Träger durch Aufhängungsmittel aufgehängt ist, welche den Resonator thermisch isolieren, und dadurch, dass der optische Resonator (8) dazu bestimmt ist, in einer gasförmigen Umgebung aufgehängt zu werden, und dadurch, dass die Heizmittel (20) in einem Abstand von dem optischen Resonator (8) angeordnet sind.

**2.** Wärmeflusssensor nach Anspruch 1, wobei der Abstand, der die Heizmittel (20) und den optischen Resonator (8) trennt, zwischen 200 nm und 10 $\mu$m liegt.

**3.** Wärmeflusssensor nach Anspruch 1 oder 2, wobei die Heizmittel (20) Joule-Effekt-Heizmittel sind.

**4.** Wärmeflusssensor nach Anspruch 1, 2 oder 3, wobei die Heizmittel (20) mindestens einen leitfähigen Draht umfassen, der an dem Träger aufgehängt ist, wobei der Draht mit einer elektrischen Spannungsquelle oder elektrischen Stromquelle verbunden ist, oder wobei die Heizmittel (20') mindestens eine Schicht aus einem elektrisch resistiven Material umfassen, die gegenüber dem optischen Resonator (8) liegt und auf dem Träger angeordnet ist, wobei die Schicht aus elektrisch resistivem Material mit einer elektrischen Spannungsquelle oder elektrischen Stromquelle verbunden ist.

5. Wärmeflusssensor nach Anspruch 1, wobei die Heizmittel (30) eine Lichtstrahlquelle umfassen, die konfiguriert ist, um einen Lichtstrahl in den optischen Resonator (8) zu injizieren, und deren Leistung eine Eigenerwärmung des optischen Resonators (8) gewährleistet.

6. Wärmeflusssensor nach einem der Ansprüche 1 bis 5, wobei die Aufhängungsmittel mindestens einen Tragbalken (12) umfassen, wobei der Tragbalken (12) vorteilhafterweise eine Breite zwischen 50 nm und 10 $\mu$m für eine Dicke zwischen 50 nm und 500 nm und eine Länge zwischen 1 $\mu$m und 100 $\mu$m aufweist.

7. Wärmeflusssensor nach einem der Ansprüche 1 bis 6, wobei der optische Resonator (8) eine optische Scheibe, einen photonischen Kristall oder einen optischen Ring mit einem Radius beispielsweise zwischen 1 $\mu$m und 100 $\mu$m umfasst.

8. Wärmeflusssensor nach einem der Ansprüche 1 bis 7, wobei das erste Mittel zum Injizieren eines Messlichtstrahls in den optischen Resonator (8) ein Wellenleiter ist, und das zweite Mittel zum Sammeln durch den gleichen Wellenleiter gebildet ist, wie derjenige des ersten Mittels, wobei ein reflektierter Lichtstrahl gesammelt wird.

9. Wärmeflusssensor nach einem der Ansprüche 1 bis 7, wobei das erste Mittel zum Injizieren eines Messlichtstrahls in den optischen Resonator (8) ein Wellenleiter ist, und wobei das zweite Mittel zum Sammeln durch einen anderen Wellenleiter gebildet ist, wobei ein durchgelassener Lichtstrahl gesammelt wird.

10. Wärmeflusssensor nach einem der Ansprüche 1 bis 7, wobei das erste Mittel zum Injizieren eines Messlichtstrahls in den optischen Resonator (8) ein Wellenleiter ist, und wobei das zweite Mittel zum Sammeln, welches einen reflektierten Lichtstrahl sammelt, durch den Wellenleiter des ersten Mittels gebildet ist, und wobei der Sensor auch einen anderen Wellenleiter (18) aufweist, der einen durchgelassenen Lichtstrahl sammelt.

11. Gassensor, der mindestens einen Wärmeflusssensor nach einem der Ansprüche 1 bis 10 umfasst, wobei der optische Resonator (8) in einem Fluidkanal (2) zwischen einem Einlass für die Versorgung des Fluidkanals mit einem Gasgemisch und einem Auslass zum Ableiten des Gasgemischs aus dem Fluidkanal (2) aufgehängt ist.

12. Sensor, welcher ein Pirani-Messgerät bildet, umfassend wenigstens einen Wärmeflusssensor nach einem der Ansprüche 1 bis 10, wobei der optische Resonator (8) in einem gasförmigen Medium aufgehängt ist, dessen Druck gemessen werden soll.

13. Messsystem, umfassend mindestens einen Sensor nach einem der Ansprüche 1 bis 12, einen Emitter (22) eines Messlichtstrahls, der mit dem ersten Mittel verbunden ist, mindestens einen mit dem zweiten Mittel verbundenen Fotodetektor (24) und eine Verarbeitungselektronik (28) für die von dem Fotodetektor abgegebenen Signale, wobei der Emitter (22) vorteilhafterweise einen amplitudenmodulierten Strahl emittiert, oder/und wobei der Emitter (22) ein Laser ist.

14. Messsystem nach Anspruch 13 in Kombination mit Anspruch 5, wobei der Emitter (22) auch den Emitter des Lichtstrahls bildet, welcher die Eigenerwärmung des optischen Resonators ermöglicht.

15. Messsystem nach Anspruch 13 oder 14, umfassend einen anderen Gassensor nach Anspruch 11, wobei das Gasgemisch ein Trägergas und mindestens einen zu detektierenden Analyten umfasst, wobei der Fluidkanal von dem einen der Gassensoren (C2) dazu bestimmt ist, mit Gasgemisch versorgt zu werden, und wobei der Fluidkanal des anderen Gassensors (C1) nur mit Trägergas versorgt wird, wobei das zweite Mittel zum Sammeln jedes Gassensors über einen Fotodetektor mit der Verarbeitungselektronik verbunden ist, um eine differentielle Messung zu ermöglichen.

16. Messsystem nach Anspruch 13 oder 14, umfassend einen anderen Gassensor nach Anspruch 11, und wobei das Gasgemisch ein Trägergas und mindestens einen zu detektierenden Analyten umfasst, wobei der Fluidkanal von dem einen der Gassensoren (C2) mit Gasgemisch versorgt wird und der Fluidkanal des anderen Gassensors (C1) nur mit Trägergas versorgt wird, wobei das zweite Mittel zum Sammeln jedes Gassensors über eine Vorrichtung zur Rückgewinnung und Interferenz der Messlichtstrahlen von den zwei Gassensoren mit der Verarbeitungselektronik verbunden ist, um die Phasenverschiebung zwischen den von den beiden zweiten Mitteln gesammelten Detektionsstrahlen zu messen, um eine Interferenzmessung zu ermöglichen.

**Claims**

1. Heat flux sensor comprising at least one optical resonator (8); a support, said optical resonator (8) comprising at least one waveguide, at least one introduction means (16) configured to introduce a measurement light beam into the waveguide, at least one collection means (16, 18) configured to collect a detection light beam coming from the optical resonator waveguide and means for heating (20, 20' 30) said optical resonator (8), **characterized in that** the waveguide is suspended on the support by suspension means which isolate thermally the resonator and that said optical resonator (8) is configured to be suspended in a gaseous environment and **in that** the heating means (20) are located away from the optical resonator (8).

2. Heat flux sensor according to claim 1, wherein the distance separating the heating means (20) and the optical resonator (8) being advantageously between 200 nm and 10 $\mu$m.

3. Heat flux sensor according to claim 1 or 2, wherein the heating means (20) are Joule effect heating means.

4. Heat flux sensor according to claim 1, 2 or 3, wherein the heating means (20) comprise at least one conductive wire suspended on said support, the wire being connected to a source of electrical voltage or of electrical current, or wherein the heating means (20') comprise at least one layer of electrical resistance material located facing the optical resonator (8) and arranged on the support, the layer of electrical resistance material being connected to a source of electrical voltage or of electrical current.

5. Heat flux sensor according to claim 1, wherein the heating means (30) comprise a light beam source configured to introduce a light beam into the optical resonator (8) and whose power causes self-heating of the optical resonator (8).

6. Heat flux sensor according to one of claims 1 to 5, wherein the suspension means comprise at least one beam (12), the beam (12) having advantageously a width between 50 nm and 10 $\mu$m with a thickness of between 50 nm and 500 nm and a length between 1 $\mu$m and 100 $\mu$m.

7. Heat flux sensor according to one of claims 1 to 6, wherein the optical resonator (8) comprises an optical disk, a photonic crystal or an optical ring having a radius for example between 1 $\mu$m and 100 $\mu$m

8. Heat flux sensor according to one of claims 1 to 7, wherein the introduction means is a waveguide and the collecting means is formed of the same waveguide as that of the introduction means, said collecting means collecting a reflected light beam.

9. Heat flux sensor according to one of claims 1 to 7, wherein the introduction means is a waveguide and s collecting means is formed of another waveguide, said c collecting means collecting a transmitted light beam.

10. Heat flux sensor according to one of claims 1 to 7, wherein the introduction means is a waveguide and the collecting means collecting a reflected light beam is formed by the waveguide of the first means, and wherein said sensor also comprises another waveguide (18) collecting a transmitted light beam.

11. Gas sensor comprising at least one thermal flux sensor according to claim 1 to 10, wherein the optical resonator (8) is suspended in a fluid channel (2) between an input supplying a gas mixture to the fluid channel and an output removing said gas mixture from the fluid channel (2).

12. Sensor forming a Pirani gauge comprising at least one heat flux sensor according to one of claims 1 to 10 wherein the optical resonator (8) is suspended in a gas mixture whose pressure it is sought to measure.

13. Measurement system comprising at least one sensor according to one of claims 1 to 12, a measurement light beam emitter (22) of a measurement light beam connected to the introduction means, at least one photodetector (24) connected to the collector means and electronics (28) for processing the signals emitted by the photodetector, the emitter (22) advantageously emitting an amplitude-modulated beam, and/or the emitter (22) is a laser.

14. Measurement system according to claim 13 in combination with claim 5, wherein the emitter (22) also forms a light beam emitter allowing self-heating of the optical resonator.

15. Measurement system according to claim 13 or 15, comprising another gas sensor according to claim 11, wherein the gas mixture comprises a carrier gas and at least one analyte to be detected, the fluid channel of one of the gas sensors (C2) being intended to be supplied with a gas mixture and the fluid channel of the other gas sensor (C1) being intended to be supplied with the carrier gas alone, the collecting means of each gas sensor being connected via a photodetector to the processing electronics in order to allow a differential measurement to be made.

16. Measurement system according to claim 13 or 14, comprising another gas sensor according to claim 11, and wherein the gas mixture comprises a carrier gas and at least one analyte to be detected, the fluid channel of one of the gas sensors (C2) being supplied with a gas mixture and the fluid channel of the other gas sensor (C1) being supplied with the carrier gas alone, the collecting means of each gas sensor being connected, via a recovery and interference device for the measurement light beams emerging from the two gas sensors, to the processing electronics in order to allow the phase-shift between the detection beams collected by the two collecting means to be measured so as to allow an interference measurement to be made.

FIG.1

FIG.2

FIG.3A

FIG.3B

FIG.4

FIG.5

FIG.6

FIG.7

FIG.8A

FIG.8B

FIG.9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011044547 A

**Littérature non-brevet citée dans la description**

- **F.T. ZHANG et al.** A micro-Pirani vacuum gauge based on a micro-hotplate technology. *Sensors & Actuators A,* 2006, vol. 126, 300-305

- **DAVID C AVELINE et al.** Miniature trace gas detector based on microfabricated optical resonators. *Technology Focus : Sensors,* Octobre 2013, 5-6